# EUROPÄISCHE PATENTSCHRIFT

(11) **EP 0 399 243 B1**
(45) Veröffentlichungstag und Bekanntmachung des Hinweises auf die Patenterteilung: **30.11.1994**
(21) Anmeldenummer: 90108026.7
(22) Anmeldetag: 27.04.1990
(51) Int. Cl.: B65H 54/38

(54) **Verfahren und Vorrichtung zum Vermeiden von Bildwicklungen beim Wickeln einer Kreuzspule**
Method and apparatus for avoiding pattern windings during the winding of a cross-wound bobbin
Procédé et dispositif pour éviter l'enroulage en ruban lors du bobinage d'une bobine croisée

(30) Priorität: 24.05.1989 DE 3916918
(43) Veröffentlichungstag der Anmeldung: 28.11.1990
(73) Patentinhaber: W. SCHLAFHORST AG & CO., D-41061 Mönchengladbach (DE)
(72) Erfinder: Hermanns, Ferdinand-Josef, D-5140 Erkelenz (DE)
(74) Vertreter: Hamann, Arndt, Dipl.-Ing.

(56) Entgegenhaltungen:
- EP-A- 0 016 714
- DE-A- 2 403 341
- DE-A- 2 914 924
- DE-A- 3 521 152
- DE-A- 3 703 869
- DE-A- 3 727 930
- DE-A- 3 822 358
- US-A- 3 910 514

## Beschreibung

Die Erfindung betrifft ein Verfahren und eine Vorrichtung zum Vermeiden von Bildwicklungen beim Wickeln einer Kreuzspule gemäß dem Oberbegriff des Anspruches 1 beziehungsweise 5.

Derartige Verfahren und Vorrichtungen haben das Ziel, die während des Wickelns von Zeit zu Zeit, innerhalb bestimmter Druchmesserbereiche, auf dem Spulenumfang entstehende Bildwicklungen, das heißt eng aneinander- oder übereinanderliegende Windungen, zu verhindern.

Es ist bekannt, eine Antriebstrommel, die die Kreuzspule durch Friktionskraft antreibt, über eine periodisch einschwenkende Reibrolle mit einer Antriebswelle in zeitlich begrenzte Antriebsverbindung zu bringen. Nach einer Antriebsunterbrechung tritt beim Wiedereinschwenken der Reibrolle ein Schlupf zwischen der Antriebstrommel und der Kreuzspule auf, wodurch die entstehende Bildwicklung gestört und aufgelöst werden soll. Insbesondere dadurch, daß der Schlupf nur periodisch auftritt, wird die Bildwicklung nur ungenügend aufgelöst.

Es ist auch bekannt, während des Wickelns der Kreuzspule ein mit dieser verbundenes Element mit wechselnder Bremskraft zu bremsen. Die Bremskraft kann so eingestellt und verändert werden, daß zwischen Kreuzspule und Antriebstrommel ein Schlupf wechselnder Größe auftritt. Das rotierende Element wird dabei mit einer Bremse in Kontakt gebracht, die eine Wirkverbindung zu einem Bremskrafteinsteller aufweist. Die Bremse wird dabei relativ stark belastet, läuft heiß, und es geht Antriebsenergie verloren. Die Lebensdauer der Bremse ist stark eingeschränkt.

Die DE-A 35 21 152 beschreibt ein Verfahren und eine dazugehörige Vorrichtung zum Vermeiden von Bildwicklungen. Dabei wird die Umfangsgeschwindigkeit der Antriebstrommel fortlaufend verändert und gleichzeitig die Kreuzspule oder ein mit dieser verbundenes rotierendes Element mit variabler Bremskraft so gebremst, daß die Umfangsgeschwindigkeit der Kreuzspule innerhalb Toleranzgrenzen konstant bleibt. Dabei ist die mittlere Umfangsgeschwindigkeit der Antriebstrommel größer als die mittlere Umfangsgeschwindigkeit der Kreuzspule.

Durch die gattungsbildende DE-A 38 22 358 ist ein Antriebsverfahren für Walzen eines Spulenautomaten bekannt, bei dem mit dem Ziel einer Bildstörung die Antriebswalze abwechselnd beschleunigt und verzögert wird. Ausgehend von der an sich bekannten Tatsache, daß die beim generatorischen Betrieb des Motors in der Abbremsphase anfallende Energie nutzbar gemacht werden kann, wird jeweils gleichzeitig die Hälfte aller Spulstellen beschleunigt und die andere Hälfte gebremst, um die Bremsenergie in vollem Umfang nutzen zu können. Dies führt zwangsläufig dazu, daß sich die Bildstörung im Verlaufe der Spulenreise durch Änderung von deren Trägheit gravierend verändert. Während es bei geringem Spulendurchmesser nur zu einer geringen Bildstörung kommt, kann es aufgrund hohen Schlupfes am Ende der Spulenreise zu Fadenabschlägen kommen.

Aufgabe der Erfindung ist es, ein Verfahren und eine Vorrichtung vorzuschlagen, um mit möglichst geringem Aufwand eine hocheffektive Bildstörung zu erreichen.

Diese Aufgabe wird erfindungsgemäß durch die Merkmale der Ansprüche 1 und 5 gelöst.

Bei Anwendung der Erfindung werden Gleichlaufphasen zwischen Kreuzspule und Antriebstrommel vermieden. Im Verlauf einer Periode der periodischen Funktion, nach der die Antriebstrommel beschleunigt und verzögert wird, sind lediglich zwei Schnittpunkte des Geschwindigkeitsverlaufes der Umfangsgeschwindigkeit von Kreuzspule und Antriebstrommel vorhanden. Da es sich in diesen Schnittpunkten jeweils nur um einen momentanen Gleichlauf handelt, ist davon auszugehen, daß sich die Umfangsgeschwindigkeiten von Kreuzspule und Antriebstrommel ständig unterscheiden. Dadurch wird der Effekt der Bildstörung wesentlich erhöht.

Durch die Änderung der Frequenz und/oder Amplitude der periodischen Funktionen der Veränderung der Umfangsgeschwindigkeit der Antriebstrommel in Abhängigkeit vom Durchmesser der Kreuzspule ist es möglich, innerhalb vorgegebener Toleranzgrenzen die Phasenverschiebung, mit der die Kreuzspule dem Bewegungsablauf der Antriebstrommel folgt, über die gesamte Spulenreise annähernd konstant zu halten. Bei Einhaltung der genannten Werte werden Fadenabschläge durch die erfindungsgemäße Bildstörung nicht hervorgerufen.

Die Erfindung gestattet es, auf aufwendige Bremsen für das Bremsen der Kreuzspule am Spulenrahmen zu verzichten.

Die Erfindung ist durch die Merkmale der Ansprüche 2 bis 4 und 6 bis 13 vorteilhaft weitergebildet.

Durch die Anordnung eines Zwischenkreises, in den die Bremsenergie, die in der Abbremsphase entsteht, wieder einspeisbar ist, erfordert die erfindungsgemäße Bildstörung keinen erheblichen Energieverbrauch. Dieser Energieverbrauch kann noch weiter herabgesetzt werden, wenn ein Elektromotor, der mit Mehrphasenwechselstrom betrieben wird, eine für die auftretenden Beschleunigungsmomente geeignete Schlupfkennlinie aufweist.

Die Erfindung soll anhand von Ausführungsbeispielen näher erläutert werden. In den zugehörigen Zeichnungen zeigen:
- Fig. 1: eine vereinfachte Darstellung einer Spulstelle, wobei zwischen Motor und Antriebstrommel ein Drehzahlwandler angeordnet ist,
- Fig. 2: eine Variante der Vorrichtung nach Fig. 1, wobei dem Elektromotor ein Wechselrichter vorgeschaltet ist,
- Fig. 3: eine grafische Darstellung des periodischen Verlaufes der Umfangsgeschwindigkeiten von Antriebstrommel und Kreuzspule,
- Fig. 4: einen Abschnitt einer Spulmaschine mit zentralem Antrieb,
- Fig. 5: eine Seitenansicht einer Spulstelle mit Einzelantrieb für die Antriebstrommel und einem Meßwertaufnehmer für die Winkelstellung des Spulenrahmens und
- Fig. 6: eine Vorderansicht der Spulstelle nach Fig. 5.

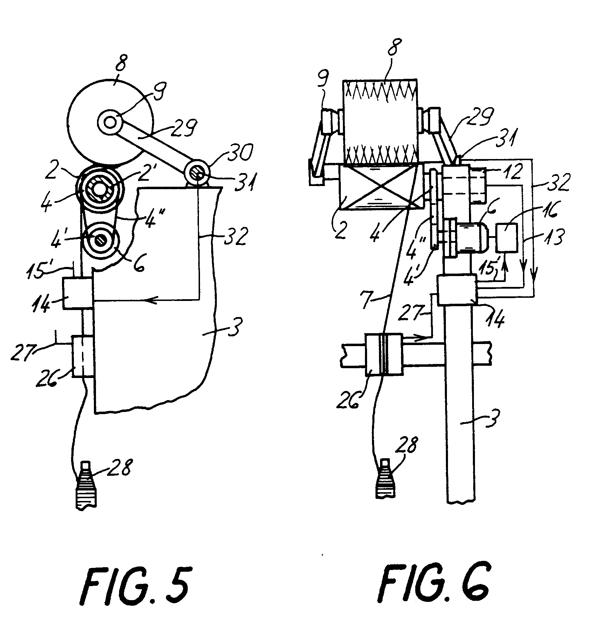

An einer Spulstelle 1 wird durch eine Antriebstrommel 2 mittels Friktion eine in einer Spulenhalterung 9 gelagerte Kreuzspule 8 angetrieben. Dabei wird ein von einem Kops 28 (Fig. 5 und 6) abgezogener Faden 7 auf die Kreuzspule 8 aufgewunden. Dieser Faden 7 wird mittels einer Kehrgewinderille 2˝ der Antriebstrommel 2 parallel zur Achse der Kreuzspule 8 verlegt. Dabei entsteht eine wilde Kreuzbewicklung.

Die Trommelwelle 2′ der Antriebstrommel 2 ist im Maschinengestell 3 gelagert. Der Antrieb der Trommelwelle 2′ erfolgt über die Riemenscheiben 4 und 4′ sowie den Keilriemen 4˝.

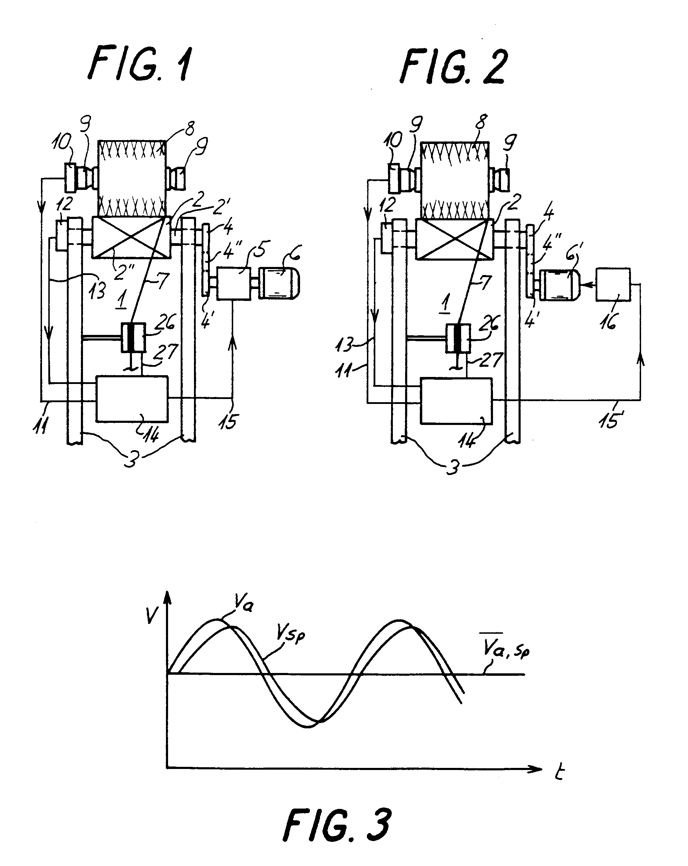

Gemäß einer ersten Variante, die in Fig. 1 dargestellt ist, ist zwischen einem Elektromotor 6 und der Riemenscheibe 4′ ein Drehzahlwandler 5 angeordnet. Dieser Drehzahlwandler 5 wird von einem Rechner 14 über eine Steuerleitung 15 gesteuert.

In den Rechner 14 wurde die periodische Grundfunktion, zum Beispiel eine Sinusfunktion, eingegeben. Während des Spulprozesses erhält der Rechner weitere Informationen von Meßwertaufnehmern 10 und 12, die an der Spulenhalterung 9 beziehungsweise der Trommelwelle 2′ angeordnet sind, über Leitungen 11 und 13. Die Meßwertaufnehmer 10 und 12 bestehen beispielsweise aus auf den Drehwinkel ansprechenden Impulsgebern. Aus dem Vergleich der Durchschnittswerte der Winkelgeschwindigkeiten von Kreuzspule 8 und Antriebstrommel 2 ermittelt der Rechner 14 den jeweiligen Durchmesser der Kreuzspule 8. Dieser Durchmesserwert korreliert zur Massenträgheit der Kreuzspule. In Abhängigkeit davon wird die eingegebene Grundfunktion im Rechner entweder in ihrer Amplitude oder in ihrer Frequenz oder auch in bei den Parametern entsprechend abgesenkt. Auf diese Weise wird die Phasenverschiebung zwischen den periodischen Funktionen der Umfangsgeschwindigkeiten von Kreuzspule 8 und Antriebstrommel 2 während der gesamten Spulenreise annähernd konstant gehalten.

Ein im Fadenverlauf des Fadens 7 angeordneter Fadenwächter 26 ist über eine Leitung 27 ebenfalls mit dem Rechner 14 verbunden. Zeigt dieser Fadenwächter 26 das Fehlen des Fadens 7, das heißt einen Fadenbruch, an, unterbricht der Rechner 14 über die Steuerleitung 15 und den Drehzahlwandler 5 die Verbindung. zwischen Elektromotor 6 und Riemenscheibe 4′.

In einer Variante der Erfindung nach Fig. 2 ist der Elektromotor 6′ direkt mit der Riemenscheibe 4′ verbunden. Dieser Elektromotor 6′, der im 4-Quadrantenbetrieb steuerbar ist, wird von einem Wechselrichter 16 gesteuert. Dieser Wechselrichter 16 erhält seine Steuerbefehle über eine Steuerleitung 15′ vom Rechner 14. Das Erzeugen der Steuersignale im Rechner 14 erfolgt analog der Variante 1. Die in der Abbremsphase frei werdende Bremsenergie wird über einen nicht dargestellten Zwischenkreis wieder eingespeist. Auf diese Weise kann die durch die Variation der Motorgeschwindigkeit zusätzlich benötigte Energie deutlich herabgesetzt werden.

Der mit Mehrphasenwechselstrom betriebene Elektromotor 6′ weist eine für die auftretenden Beschleunigungsmomente geeignete Schlupfkennlinie auf. Auch diese Maßnahme führt zu einem effektiven Energieeinsatz.

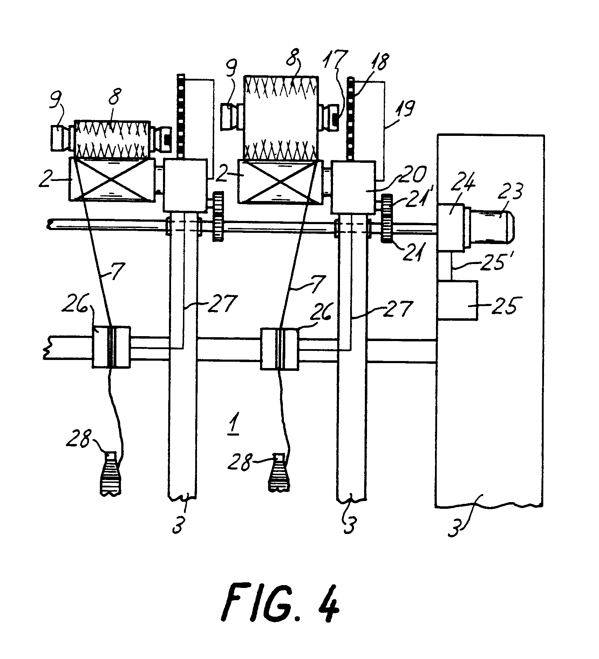

Die Anwendung der Erfindung ist nicht auf den Einzelantrieb der Spulstellen beschränkt. So ist in Fig. 4 eine Variante dargestellt, in der die Antriebstrommeln 2 der Spulmaschine über eine gemeinsame Antriebswelle 22 von einem Elektromotor 23 angetrieben werden. Bei dieser Variante der Erfindung ist zwischen Elektromotor 23 und Antriebswelle 22 ein Drehzahlwandler 24 geschaltet, der die Motordrehzahl so auf die Antriebswelle 22 überträgt, daß deren Drehzahl entsprechend der vorgegebenen Grundfunktion schwankt. Gesteuert wird der Drehzahlwandler 24 über eine Steuerleitung 25′ von einem Steuergerät 25.

Die Drehbewegung der Antriebswelle 22 wird über Antriebsräder 21 und 21′ in Steuereinheiten 20 übertragen.

In dieser Variante der Erfindung wird der Momentandurchmesser der Kreuzspule 8 durch einen Meßwertaufnehmer ermittelt, der aus einer am Spulenrahmen 29 befestigten Lichtquelle 17 und einer parallel zu dem während der Spulenreise von der Lichtquelle 17 zurückgelegten Weg befindlichen Fotozellenanordnung 18 besteht. Dabei senden die jeweils der Lichtquelle 17 gegenüberstehenden Fotozellen der Fotozellenanordnung 18 ein dem Durchmesser der Kreuzspule 8 entsprechendes Signal über die Leitung 19 an die Steuereinheit 20. In dieser Steuereinheit 20 kann zum Beispiel eine Hysteresekupplung angeordnet sein, die in Abhängigkeit von der anliegenden Spannung die Schwankungen der Drehzahl, die von der Antriebswelle 22 eingebracht wurden, unterschiedlich stark überträgt. Damit verringert sich mit zunehmendem Durchmesser der Kreuzspule 8 die Amplitude der übertragenen periodischen Funktion der Änderung der Drehzahl beziehungsweise der Umfangsgeschwindigkeit der Antriebstrommel 2. Außerdem wird analog den bisherigen Varianten bei Fadenbruch, der vom Fadenwächter 26 erfaßt wird, über die Leitung 27 eine nicht dargestellte Kupplung innerhalb der Steuereinheit 20 betätigt.

Eine weitere Variante der Erfassung des jeweiligen Momentandurchmessers der Kreuzspulen 8 ist in den Fig. 5 und 6 dargestellt. Dabei wird die Winkelstellung des Spulenrahmens 29, der in der Spulenrahmenachse 30 gelagert ist, durch einen Meßwertaufnehmer 31 erfaßt, der zum Beispiel aus einem Drehwiderstand bestehen kann. Die der Winkelstellung des Spulenrahmens 29 entsprechenden Spannungswerte werden über eine Leitung 32 in den Rechner 14 übertragen. Ebenso ist auch eine direkte Übertragung in die Steuereinheit 20 zur Betätigung der Hysteresekupplung möglich.

Erfolgt die Steuerung durch den Rechner 14, kann dieser bei Erreichen von Hauptbildzonen, für die die jeweiligen Durchmesserwerte eingegeben wurden, bis zum Verlassen dieser Hauptbildzonen die Amplitude und/oder Frequenz der periodischen Funktion der Veränderung der Umfangsgeschwindigkeit der Antriebstrommel 2 erhöhen.

Das in Fig. 3 dargestellte Diagramm verdeutlicht den sinusförmigen Verlauf der Umfangsgeschwindigkeiten der Antriebstrommel 2 (Va) und der Kreuzspule 8 (Vsp) in Abhängigkeit von der Zeit (t) . Weiterhin ist aus dieser grafischen Darstellung zu erkennen, daß die Kreuzspule 8 dem Bewegungsablauf der Antriebstrommel 2 permanent phasenverschoben folgt. Weiterhin ist zu ersehen, daß innerhalb einer Periode lediglich zwei Schnittstellen der Umfangsgeschwindigkeiten auftreten. Demzufolge ist der Gleichlauf zwischen Antriebstrommel 2 und Kreuzspule 8 auf diese Schnittpunkte beschränkt. Dadurch wird eine äußerst effektive Bildauflösung erreicht. Die mittleren Umfangsgeschwindigkeiten der Antriebstrommel 2 und der Kreuzspule 8 sind dabei gleich (V̅a,sp).

Da ein relativ hoher Wirkungsgrad der Bildauflösung bei Anwendung der Erfindung erreicht wird, ist es möglich mit relativ niedrigen Beschleunigungswerten zu arbeiten. Bei einer Frequenz der periodischen Funktion des Bewegungsablaufes der Antriebstrommel 2 im Bereich von 1 Hz kann sowohl eine effektive Bildstörung erreicht als auch ei ne zu starke Fadenbeanspruchung der jeweils oberen Fadenlagen der Kreuzspule 8 vermieden werden. Die Phasenverschiebung hängt neben den Parametern der periodischen Funktion der Veränderung der Umfangsgeschwindigkeit der Antriebswalze und dem Durchmesser der Kreuzspule 8 auch vom aufgespulten Faden, insbesondere dessen Rauhigkeit ab. Bei relativ glatten Fäden erreicht man deshalb die gleiche Phasenverschiebung bereits bei geringerer Amplitude der oben genannten periodischen Funktion. Im Normalfall sollte diese Amplitude 12 % der mittleren Umfangsgeschwindigkeit nicht überschreiten, da es sonst in der Abbremsphase zu Fadenabschlägen an den Seiten der Kreuzspulen 8 kommen könnte. Bei Einhaltung der genannten Werte werden derartige Fadenabschläge durch die erfindungsgemäße Bildstörung nicht hervorgerufen.

## Patentansprüche

1. Verfahren zum Vermeiden von Bildwicklungen beim Wickeln einer Kreuzspule, die durch eine mit Kehrgewinderillen für die Fadenführung versehene Antriebstrommel angetrieben wird, wobei die Umfangsgeschwindigkeit der Antriebstrommel sich ständig ändert, die Kreuzspule (8) ausschließlich durch die Antriebstrommel (2) beschleunigt und verzögert wird und die Antriebstrommel (2) nach einer vorgebbaren periodischen Funktion durch ihren Antrieb sowohl beschleunigt als auch abgebremst wird,
dadurch gekennzeichnet,
daß die Kreuzspule (8) dem Bewegungsablauf der Antriebstrommel (2) permanent phasenverschoben folgt, daß der Spulendurchmesser der Kreuzspule (8) ständig gemessen wird und die Frequenz und/oder Amplitude der periodischen Funktion der Veränderung der Umfangsgeschwindigkeit der Antriebstrommel (2) so geändert werden, daß ein vorgebbarer Wert der Phasenverschiebung nicht überschritten wird.

2. Verfahren nach Anspruch 1, dadurch gekennzeichnet, daß die mittleren Umfangsgeschwindigkeiten der Antriebstrommel (2) und der Kreuzspule (8) im wesentlichen gleich gehalten werden.

3. Verfahren nach einem der Ansprüch 1 oder 2, dadurch gekennzeichnet, daß der sich während des Herstellens der Kreuzspule ändernde Durchmesser der Kreuzspule (8) durch das jeweilige Verhältnis der mittleren Winkelgeschwindigkeiten der Kreuzspule und der Antriebstrommel (2) ermittelt wird.

4. Verfahren nach einem der Ansprüche 1 bis 3, dadurch gekennzeichnet, daß die Amplitude und/oder die Frequenz der periodischen Funktion der Veränderung der Umfangsgeschwindigkeit der Antriebstrommel (2) in den bekannten kritischen Bereichen, in denen Bildwicklungen entstehen, den sogenannten Hauptbildzonen, erhöht werden.

5. Vorrichtung zum Vermeiden von Bildwicklungen beim Wickeln einer Kreuzspule, die durch eine mit Kehrgewinderillen (2'') für die Fadenführung versehene Antriebstrommel (2) antreibbar ist, zur Durchführung des Verfahrens nach Anspruch 1, wobei der Antrieb der Antriebstrommel (2) Mittel (5; 16; 20, 24) besitzt, um die Antriebstrommel (2) zu beschleunigen und abzubremsen, dadurch gekennzeichnet, daß Meßwertaufnehmer (10, 12; 17, 18; 31) zur Aufnahme von den Spulendurchmesser der Kreuzspule repräsentierenden Meßwerten vorgesehen sind und daß eine Befehlsverbindung (15; 15'; 19; 32) vorhanden ist, die den Antrieb in Abhängigkeit vom ermittelten Durchmesser über geeignete Mittel (5; 10; 20, 24) in der Amplitude und/oder Frequenz der periodischen Funktion der Veränderung der Umfangsgeschwindigkeit der Antriebstrommel (2) steuert .

6. Vorrichtung nach Anspruch 5, dadurch gekennzeichnet, daß die Meßwertaufnehmer (10, 12) für die Erfassung der momentanen Winkelgeschwindigkeit der Antriebstrommel (2) und der Kreuzspule (8) vorgesehen sind, wobei diese Meßwertaufnehmer Wirkverbindungen zu einem Rechner (14) oder Mikroprozessor aufweisen und daß der Rechner (14), der aus dem Verhältnis der Durchschnittswerte der Winkelgeschwindigkeiten den Durchmesser der Kreuzspule (8) ermittelt.

7. Vorrichtung nach Anspruch 5, dadurch gekennzeichnet, daß ein Meßwertaufnehmer (17, 18; 31) am Spulenrahmen (29) für das Erfassen der dem Spulendurchmesser proportionalen Winkelstellung des Spulenrahmens (29) angeordnet ist.

8. Vorrichtung nach einem der Ansprüche 5 bis 7, dadurch gekennzeichnet, daß der Antrieb der Antriebstrommel (2) durch den Rechner (14) über die Befehlsverbindung (15, 15') so steuerbar ist, daß bei Erreichen von aus dem ermittelten Durchmesser der Kreuzspule (8) erkannten Hauptbildzonen die Amplitude und/oder Frequenz der periodischen Funktion der Veränderung der Umfangsgeschwindigkeit der Antriebstrommel (2) zeitweise erhöhen.

9. Vorrichtung nach einem der Ansprüche 5 bis 8, dadurch gekennzeichnet, daß zwischen Elektromotor (23) und Antriebstrommel (2) eine Hysteresekupplung für die Steuerung der Amplitude der periodischen Funktion der Veränderung der Umfangsgeschwindigkeit der Antriebstrommel angeordnet ist.

10. Vorrichtung nach Anspruch 5, dadurch gekennzeichnet, daß der Antrieb der Antriebstrommel (2) einen im 4-Quadrantenbetrieb steuerbaren Elektromotor 6' besitzt.

11. Vorrichtung nach Anspruch 5,dadurch gekennzeichnet, daß ein Zwischenkreis vorhanden ist, in den die Bremsenergie, die in der Abbremsphase entsteht, wieder einspeisbar ist.

12. Vorrichtung nach Anspruch 5, dadurch gekennzeichnet, daß ein Elektromotor (6') eingesetzt wird, der mit Mehrphasenwechselstrom betrieben wird und eine für die auftretenden Beschleunigungsmomente geeignete Schlupfkennlinie aufweist.

13. Vorrichtung nach Anspruch 5, dadurch gekennzeichnet, daß zwischen Elektromotor (6) und Antriebstrommel (2) ein Drehzahlwandler (5; 24) angeordnet ist.

## Claims

1. Process for preventing pattern winding when winding a cross-wound bobbin, which is driven by a drive pulley provided with inverted screw thread grooves for the thread guide, wherein the circumferential speed of the drive pulley constantly changes, the cross-wound bobbin (8) is accelerated and delayed solely by the drive pulley (2), and the drive pulley (2) is both accelerated and braked by its drive according to a predetermined periodic function, characterised in that the cross-wound bobbin (8) follows the movement cycle of the drive pulley (2) permanently out-of-phase, that the bobbin diameter of the cross-wound bobbin (8) is constantly measured and the frequency and/or amplitude of the periodic function for changing the circumferential speed of the drive pulley (2) are changed to such an extent that a predetermined value for the phase shift is not exceeded.

2. Process according to Claim 1, characterised in that the average circumferential speeds of the drive pulley (2) and the cross-wound bobbin (8) are kept substantially constant.

3. Process according to one of Claims 1 or 2, characterised in that the diameter of the cross-wound bobbin (8), which changes during manufacture of the cross-wound bobbin is determined by the respective ratio of the average angular speeds of the cross-wound bobbin and the drive pulley (2).

4. Process according to one of Claims 1 to 3, characterised in that the amplitude and/or the frequency of the periodic function for changing the circumferential speed of the drive pulley (2) are increased in the known critical ranges, in which pattern winding occurs, i.e. the so-called main pattern zones.

5. Device for preventing pattern winding when winding a cross-wound bobbin, which may be driven by a drive pulley (2) provided with inverted screw thread grooves (2'') for the thread guide, to implement the process according to Claim 1, wherein the drive of the drive pulley (2) has means (5; 16; 20, 24) for accelerating and braking the drive pulley (2), characterised in that transducers (10, 12; 17, 18; 31) are provided to record the measured values representing the bobbin diameter of the cross-wound bobbin, and that an instruction connection (15; 15'; 19; 32) is provided which controls the drive in dependence on the determined diameter by suitable means (5; 10; 20, 24) in the amplitude and/or frequency of the periodic function for changing the circumferential speed of the drive pulley (2).

6. Device according to Claim 5, characterised in that the transducers (10, 12) are provided to detect the current angular speed of the drive pulley (2) and the cross-wound bobbin (8), these transducers having active connections to a computer (14) or a microprocessor, and that the computer (14) determines the diameter of the cross-wound bobbin (8) from the ratio of the mean values of the angular speeds.

7. Device according to Claim 5, characterised in that a transducer (17, 18; 31) is arranged on the bobbin frame (29) to detect the angle position of the bobbin frame (29) proportional to the bobbin diameter.

8. Device according to one of Claims 5 to 7, characterised in that the drive of the drive pulley (2) may be controlled by the computer (14) via the instruction connection (15, 15') in such a manner that when main pattern zones recognised from the determined diameter of the cross-wound bobbin (8) are reached, the amplitude and/or frequency of the periodic function for changing the circumferential speed of the drive pulley (2) increase intermittently.

9. Device according to one of Claims 5 to 8, characterised in that a hysteresis coupling is arranged between the electric motor (23) and the drive pulley (2) for control of the amplitude of the periodic function for changing the circumferential speed of the drive pulley.

10. Device according to Claim 5, characterised in that the drive of the drive pulley (2) has an electric motor (6') which can be controlled in 4-quadrant operation.

11. Device according to Claim 5, characterised in that an intermediate circuit is provided, into which the braking energy produced in the braking phase may be fed back.

12. Device according to Claim 5, characterised in that an electric motor (6') is used, which is operated with multiphase alternating current and has a slippage characteristic curve suitable for the arising moments of acceleration.

13. Device according to Claim 5, characterised in that a variable speed gear (5; 24) is provided between the electric motor (6) and the drive pulley (2).

## Revendications

1. Procédé pour éviter les enroulements coïncidents lors du bobinage d'une bobine croisée qui est entraînée par un tambour d'entraînement pourvu de fentes de balayage pour le guidage du fil, cependant que la vitesse périphérique du tambour d'entraînement varie continûment, que la bobine croisée (8) est exclusivement accélérée et ralentie par le tambour d'entraînement (2), et que le tambour d'entraînement (2) est aussi bien accéléré que freiné par son entraînement selon une fonction périodique que l'on peut prédéterminer, caractérisé par le fait que la bobine croisée (8) suit avec un décalage de phase permanent la courbe du déplacement du tambour d'entraînement (2), et par le fait que l'on mesure en permanence le diamètre de la bobine croisée (8), et que l'on modifie la fréquence et/ou l'amplitude de la fonction périodique de modification de la vitesse périphérique du tambour d'entraînement (2) d'une manière telle qu'une valeur du décalage de la phase que l'on peut prédéterminer ne soit pas dépassée.

2. Procédé selon la revendication 1, caractérisé par le fait que l'on maintient égales, pour l'essentiel, les vitesses périphériques moyennes du tambour d'entraînement (2) et de la bobine croisée (8).

3. Procédé selon l'une des revendications 1 et 2, caractérisé par le fait que l'on détermine le diamètre de la bobine croisée (8), lequel varie pendant la confection de la bobine croisée, au moyen du rapport respectif entre les vitesses angulaires moyennes de la bobine croisée et du tambour d'entraînement (2).

4. Procédé selon l'une des revendications 1 à 3, caractérisé par le fait que l'on augmente l'amplitude et/ou la fréquence de la fonction périodique de modification de la vitesse périphérique du tambour d'entraînement (2) dans les zones critiques connues dans lesquelles des enroulements coïncidents prennent naissance et que l'on appelle des zones principales de coïncidence.

5. Dispositif pour éviter les enroulements coïncidents lors du bobinage d'une bobine croisée qui peut être entraînée par un tambour d'entraînement (2) pourvu de fentes de balayage (2'') pour le guidage du fil, pour la mise en oeuvre du procédé selon la revendication 1, l'entraînement du tambour d'entraînement (2) comportant des moyens (5 ; 16 ; 20, 24) pour accélérer et freiner le tambour d'entraînement (2), caractérisé par le fait qu'il est prévu des capteurs de valeurs mesurées (10, 12 ; 17, 18 ; 31) destinés à capter des valeurs mesurées représentatives du diamètre de la bobine croisée, et par le fait qu'il existe une liaison de commande (15 ; 15' ; 19 ; 32) qui, en fonction du diamètre déterminé, agit sur l'entraînement par l'intermédiaire de moyens appropriés (5 ; 16 ; 20, 24) en modifiant l'amplitude et/ou la fréquence de la fonction périodique de modification de la vitesse périphérique du tambour d'entraînement (2).

6. Dispositif selon la revendication 5, caractérisé par le fait que les capteurs de valeurs mesurées (10, 12) sont prévus pour déterminer la vitesse angulaire instantanée du tambour d'entraînement (2) et de la bobine croisée (8), ces capteurs de valeurs mesurées comportant des liaisons fonctionnelles avec un calculateur (14) ou avec un microprocesseur, et par le fait que le calculateur (14) détermine le diamètre de la bobine croisée (8) à partir du rapport entre les valeurs moyennes des vitesses angulaires.

7. Dispositif selon la revendication 5, caractérisé par le fait qu'un capteur de valeurs mesurées (17, 18 ; 31) est disposé sur le support (29) de la bobine pour capter la position angulaire du support (29) de la bobine qui est proportionnelle au diamètre de la bobine.

8. Dispositif selon l'une des revendications 5 à 7, caractérisé par le fait que l'entraînement du tambour d'entraînement (2) peut être commandé par le calculateur (14) par l'intermédiaire de la liaison de commande (15, 15') d'une manière telle que, lorsque l'on atteint les zones principales de coïncidence détectées à partir du diamètre déterminé de la bobine croisée (8), l'amplitude et/ou la fréquence de la fonction périodique de modification de la vitesse périphérique du tambour d'entraînement (2) augmente temporairement.

9. Dispositif selon l'une des revendications 5 à 8, caractérisé par le fait qu'un couplage à hystérésis destiné à agir sur l'amplitude de la fonction périodique de modification de la vitesse périphérique du tambour d'entraînement est disposé entre le moteur électrique (23) et le tambour d'entraînement (2).

10. Dispositif selon la revendication 5, caractérisé par le fait que l'entraînement du tambour d'entraînement (2) comporte un moteur électrique (6') qui peut être commandé en fonctionnement à quatre quadrants.

11. Dispositif selon la revendication 5, caractérisé par le fait qu'il est prévu un circuit intermédiaire dans lequel l'énergie de freinage qui prend naissance lors de la phase de freinage peut être à nouveau injectée.

12. Dispositif selon la revendication 5, caractérisé par le fait que l'on utilise un moteur électrique (6') qui fonctionne sur un courant alternatif polyphasé et qui présente une caractéristique de glissement convenant aux couples d'accélération qui interviennent.

13. Dispositif selon la revendication 5, caractérisé par le fait qu'un convertisseur de vitesse de rotation (5 ; 24) est disposé entre le moteur électrique (6) et le tambour d'entraînement (2).
